# EUROPEAN PATENT SPECIFICATION

(11) **EP 0 651 637 B1**
(45) Date of publication and mention of the grant of the patent: **04.10.2006**
(21) Application number: 93918261.4
(22) Date of filing: 20.07.1993
(51) Int. Cl.: A61K 31/138, A61K 31/5415, A61P 17/02

(54) **CONTROL OF WOUND SCAR PRODUCTION WITH CALMODULIN INHIBITORS OR PROTEIN KINASE C INHIBITORS**
KONTROLLE DER WUNDVERNARBUNG MIT CALMODULIN-INHIBITOREN ODER PROTEIN-KINASE-C-INHIBITOREN
PROCEDE POUR DIMINUER LES CICATRICES CONSECUTIVES A DES BLESSURES A L'AIDE D'ANTAGONISTES DU CALMODULIN OU D'INHIBITEURS DE LA PROTEINE KINASE C

(30) Priority: 20.07.1992 US 916412
(43) Date of publication of application: 10.05.1995
(73) Proprietor: MASSACHUSETTS INSTITUTE OF TECHNOLOGY, Cambridge, MA 02139 (US); Arch Development Corporation, Chicago, IL 60606 (US)
(72) Inventor: LEE, Raphael C., Chicago, IL 60615 (US)
(74) Representative: Kirkham, Nicholas Andrew
(86) International application number: PCT/US1993/006804
(87) International publication number: WO 1994/002130

(56) References cited:
- WO-A-91/01624
- J. SURG. RES. vol. 49, no. 5, 1990, pages 463 - 466 R.C. LEE ET AL. 'Calcium antagonists retard extracellular matrix production in connective tissue equivalent'
- RETINA vol. 5, no. 1, 1985, pages 47 - 60 F.M. VAN BOCKXMEER ET AL. 'Models for assessing scar tissue inhibitors'
- INT. J. COLORECTAL DIS. vol. 4, no. 1, 1989, pages 30 - 36 A.M. LOTFI ET AL. 'Mesenteric fibromatosis complicating familial adenomatous polyposis: predisposing factors and results of treatment'
- ANN. CHIR. PLAST. vol. 15, no. 1, 1970, pages 54 - 56 E. MASSOUD 'Traitement des chéloides et des cicatrices hypertrophiques par la triamcinolone acétonide'
- R. BERKOW ET AL. 'The Merck Manual' 1987 , MERCK SHARP & DOHME RESEARCH LABORATORIES , RAHWAY, N.J.
- CANCER CHEMOTHER. PHARMACOL. vol. 18, no. 1, 1986, pages 17 - 20 A. LIPTON ET AL. 'Calcium antagonism by the antioestrogen tamoxifen.'
- J. SURG. RES. vol. 42, no. 5, 1987, pages 560 - 564 M.D. MCGONIGAL 'The effects of breast cancer chemotherapy on wound healing in the rat'

## Description

### Background of the Invention

The ability to heal by forming scars is essential for mammalian systems to survive wounding after injury. Normally, wound healing is a continuous process extending over a one-to-two-year period. The process can be conceptually divided into three fundamentally distinct stages. The first stage is an intensely degradative phase called the inflammatory stage. It occurs immediately after injury and provides a means to remove the damaged tissues and foreign matter from the wound. Two-to-three days later, as fibroblasts from the surrounding tissue move into the wound, the repairing process enters its second stage, the proliferation and matrix synthesis stage. The fibroblasts in the wound proliferate and actively produce macromolecules, such as collagen and proteoglycans, which are secreted into the extracellular matrix. The newly-synthesized collagen fibrils are crosslinked by lysyl oxidase and provide structural integrity to the wound. During this stage, fibroblasts also contract the intact collagen in order to reduce the surface area of the wound. This second stage lasts about three weeks. In the final, remodeling stage, the previous randomly-organized collagen fibril is aligned in the direction of mechanical tension and becomes more organized so that the mechanical strength of the wound area can be increased. The repair process is accomplished when the chemical and physical barrier functions of the skin are restored.

Normal wound healing follows a well-regulated course. However, imbalances may cause abnormal scars to form. For example, if the biosynthetic phase continues longer than necessary or degradation of collagen decreases, hypertrophic scars may form. These scars cause problems ranging from aesthetic deformity to severe limitation of motion. Hypertrophic scars more frequently occur among children and adolescents, suggesting that growth factors may influence the development of this type of scar. Hypertrophic scars are especially common in patients who have burns or wounds that heal by secondary intention. Another type of excess scar is the keloid. In this disorder, the cells appear to lack sensitivity to normal feedback signals. They are larger than hypertrophic scars and grow in an unregulated way, tending to invade normal tissue surrounding the wound. They rarely disappear spontaneously and often recur after surgical excision. The management of these scars remains a major unsolved clinical problem.

Existing therapy for hypertrophic scars and keloids includes surgery, mechanical pressure, steroids, x-ray irradiation and cryotherapy. There are many disadvantages associated with each of these methods. Surgical removal of the scar tissue is often incomplete and can result in the development of hypertrophic scars and keloids at the incision and suture points. Steroid treatments are unpredictable and often result in depigmentation of the skin. X-ray therapy is the only predictably effective treatment to date; however, because of its potential for causing cancer, it is not generally recommended or accepted.

Compositions comprising tripeptides, tetrapeptides and pentapeptides have been shown to inhibit biosynthesis of collagen and may be used to treat diseases caused by excess accumulation of collagen (Mitsubishi Chem., Japanese Patent Nos. 52083545, July 12, 1977, and 52025768, February 25, 1977). The effects of applying silastic sheets onto the surface of hypertrophic scars was studied and shown to shrink and soften scar tissue. [Ohmori, S. Aesthetic Plastic Surgery 12: 95-99 (1988)].

WO-A-91/01624 teaches the use of calcium channel blockers, a specific class of calcium antagonists, for treating hypertrophic scars, keloids and fibromatosis. The calcium channel blockers are selected from verapamil, biologically compatible cobalt salts such as cobalt chloride, and hydropyridine compounds such as nifedipine. The chosen calcium channel blocker may be admixed with a pharmaceutically acceptable vehicle for localising the calcium channel blocker at the wound site. There is no teaching in this reference of the use of Protein Kinase C or calmodulin inhibitors for controlling scar production.

RETINA, Vol. 5, No. 1, 1985, pages 47-60 teaches an in vitro model for traction retinal detachment based on the growth of chorioretinal fibroblasts in a three dimensional lattice of collagen fibrils. The model is for the preliminary screening of the efficacy of putative inhibitors for proliferative vitreoretinopathy. Among the compounds screened by this in vitro model was trifluoperazine. The authors indicate that this compound appears unsuitable for use, for being too toxic.

ANN. de CHTRURGIE PLASTIQUE, Volume XV, No. 1, 1970, pages 54-56 discloses the "treatment of choice" for keloids and hypertrophic scars, wherein a corticosteroid is injected into the site. The corticosteroid is triamcinoline acetonide. This reference contains no teaching of administering this corticosteroid with any other drug.

The Merck Mannual of Diagnosis and Therapy, XVth Edition, 1987, Merck, Sharp & Dohme Research Laboratories at page 2306 refers to the treatment of keloids by injecting the lesion with a corticosteroid such as triamcinoline acetonide. However, the disclosure here warns that the treatment is often ineffective.

In contrast, the present invention is directed at the use of a calmodulin inhibitor such as trifluoperazine or a Protein Kinase C inhibitor such as tamoxifen, alone or in combination with a corticosteroid or glucocorticosteroid, for manufacturing medicaments for controlling or reducing scar formations.

The main- and sub-claims herein define the essential and optional features respectively of the present invention.

Use of this invention yields a method for minimizing or preventing excessive scar formation, particularly hypertrophic wound healing disorders, such as hypertrophic scars and keloids. Specifically, the method comprises administering an effective amount of a calmodulin inhibitor or a Protein Kinase C inhibitor alone or in combination with a protein synthesis inhibitor (e.g. steroids) as part of a complete therapeutic regimen, to a wound site for a period of time sufficient to minimize the scar, or to prevent the formation of a hypertrophic scar. The preferred inhibitors are trifluoperazine and tamoxifen. The steroid (if used) can be selected from the corticosteroids and glucocorticosteroids, such as triamcinolone acetonide. For similar purposes vitamin E can be used. The inhibitor is applied to the wound site, such as by injecting it directly, into a scar or topically applying it onto the wound site. If a steroid or vitamin E is used in conjunction with the calcium antagonist, the steroid can be applied subsequently to the wound site, preferably within a two week time period. The steroid is also administered directly to the wound site; it may be injected or topically applied. In whatever manner they are administered, the inhibitor and/or the steroid can be admixed with a pharmaceutically acceptable vehicle to facilitate localization of the agent to the wound site. Similarly, a non-steroidal anti-inflammatory agent can be co-administered with the inhibitor The drugs may be pat into sustained release capsules to provide continuous treatment at therapeutic doses without systemic side effects.

The present invention can be used to minimize or prevent scar formation, such as hypertrophic wounds, keloids and burn scar contractures, in humans or other mammals, particularly those individuals prone to excessive scarring. The chosen inhibitor, alone or in combination with or followed by a steroid or vitamin E, can be applied to a presently existing hypertrophic scar to reverse the scarring process and essentially eliminate the scar tissue. The present invention can also be used therapeutically to control diseases associated with excessive scarring, such as Dupuytran's disease of the hand, plantar fibrosis of the foot and various other fibromatoses.

### Brief Description of the Drawings

### Detailed Description of the Invention

Calcium antagonists, as used herein, are compounds which can interfere with Calcium transport within a cell or block/inhibit one or more events involved in the calcium cascade. Several classes of calcium antagonists, include calmodulin inhibitors, Protein Kinase c inhibitors and calcium transport blockers. Calmodulin inhibitors prevent the binding of calcium to calmodulin, thereby interrupting intracellular signal transduction, including activation of Protein Kinase C, the next event in the calcium cascade. Compounds that inhibit Protein Kinase C or other downstream events can be used. Calmodulin inhibitors include phenothiazines, such a trifluoperazine and tamoxifen (also Protein Kinase C inhibitors). Calcium transport blockers, the use of which is outside the scope of the presently - claimed invention, also called calcium entry antagonists, calcium channel antagonists or calcium channel blockers, block the action of calcium channels, which are regions of cell membranes that facilitate the transport and secretion of fluids and electrolytes such as calcium into the cell [Rasmussen, It. N.E.J. Med. 314: 1094-1101 (1986)]. Compounds included in this class are phenylalkylamine compounds, such as verapamil; polyvalent ionic salts that physically block the calcium channels, such as nickel chloride, cobalt chloride and other biologically acceptable salts of these; hydropyridine compounds, such as nifedipins; and benzothiazepine compounds, such as diltiazem. Other compounds that affect the secondary messenger pathways in cellular signal transduction may have the same or similar effect as calcium antagonists on cell shape and tissue remodeling.

The present invention stems from the discovery that calcium antagonists, which interfere with calcium metabolism or transport across the cell membrane, can inhibit exocytosis in fibroblast cells; can retard biosynthesis of collagen and sulfated glycosaminoglycans (GAG); can be used to decrease the collagen content of the extracellular matrix; and can also stimulate increased collagenase activity, leading to softening of the scar tissue. These features work together to control wound scar production; by minimizing, preventing or reversing the scarring process, depending upon the course of the disease or type of wound treated.

Exocytosis, a process involved in cellular secretion of protein, is but one mechanism affected by calcium antagonist treatment. During secretion, vesicles that contain sorted and concentrated protein pinch off from the Golgi apparatus and move toward the cell membrane at the leading edge of the cell, where they fuse with the cell membrane and release protein into the extracellular space. This process of fusion and release is known as exocytosis and is one of the essential steps in secretion of extracellular matrix macro-molecules (such as glycosaminoglycans, collagen and elastin). Many diseases and disorders are characterized by excessive biosynthesis or secretion. For example, hypertrophic wound healing disorders are characterized by over-secretion of protein and collagen. This overproduction is one factor which contributes to excessive scarring or keloid formation.

It has also been observed that calcium antagonist regulate cell shape. As described in detail in the exemplification, fibroblasts that have been treated with a calcium antagonist became more rounded than untreated fibroblasts. The treated cells were tested for viability and were found to have intact cell membranes which are indicative of viable cells. The observation that treated fibroblast cells become alteration was correlated with changes in cell programming from a biosynthetic mode (mechanism normally undertaken by untreated fibroblasts) to a degradative mode. It is believed that this change toward matrix degradation, mediated by cell shape changes, plays a role in controlling wound scar production. Thus, other compounds can be studied for their ability to regulate (up regulate or down regulate) fibroblast biosynthesis by observing their interaction with calcium antagonists.

In a preferred embodiment, wound scar content can be minimized by administering an effective amount of a calcium antagonist to a hypertrophic wound site. The calcium antagonist may be administered alone, or followed by the administering of a protein synthesis inhibitor (e.g., steroid). For example, a steroid can be co-administered with the calcium antagonist, or applied separately, preferably within a two-week interval following the application of the calcium antagonist. Treatment of the wound site with the calcium antagonist, with or without the steroid, should continue for a period of time sufficient to minimize the wound area. Suitable calcium antagonists include phenothiazines, such as trifluoperazine, and tamoxifen which are respectively examples of calmodulin and Protein Kinase C inhibitors, The amount of calcium antagonist which can be effectively administered is dependent upon the type of calcium antagonist used and the scar site to be treated, and can be ascertained by monitoring the scar site during treatment. The amount can be adjusted accordingly depending upon the response observed. Threshold effective amounts of verapamil and nifedipine, use of which is outside the scope of the claimed invention are approximately 10µM and 1mM, respectively. Steroids which may be used include, but are not limited to; corticosteroids and glucocorticosteroids, such as triamcinolone acetonide (also known as KENALOG™), and vitamin E (α-tocopherol) (Ehrlich et al. 1972. Ann. Surg. 75:235). The amount of steroid which can be affectively administered will depend upon the type of steroid used.

Hydropyridine compounds such as nifedipine, use of which is outside the scope of the claimed invention are relatively insoluble in aqueous solution. Due to their insolubility, it may be advantageous to solubilize the drug in a non-polar carrier depending upon the location of the disorder to be treated. Calcium antagonists can be administered to a wound site either alone or they can be admixed with pharmaceutically acceptable vehicles to facilitate their diffusion into the wound site. One suitable vehicle is dimethyl sulfoxide in a physiologically acceptable amount. Calcium antagonists can be incorporated into liposomes for localization of concentrated quantities of the drug, as well as for the sustained release of the drug to the wound site.

Calcium antagonists can be concentrated and incorporated into controlled release polymers as an alternative mode of administering the drug (e.g., transdermal administration). Examples of controlled release polymers have been described by Folkman and Langer, U.S. patent No. 4,391,727, issued July 5, 1983; Yolles, U.S. Patent Nos. 3,880,991 issued April 29, 1975, and 3, 887,699, issued June 3, 1975; Boswell, U.S. Patent No. 3,773,919. Preferably, biodegradable polymers will be used.

The method of administering an acceptable dose of calcium antagonise to minimize scarring is dependent upon the location of the hypertrophic wound and me extent of scarring. In particular, the calcium antagonist, either alone or in combination with a pharmaceutically acceptable vehicle, can be topically applied to the surface of the wound site; it can be injected into the wound site; or it can be incorporated into a controlled release polymer and surgically implanted in a region to be treated. Surgical implantation is advantageous for treating disorders such as cirrhosis of the liver and constrictive pericarditis. This permits the calcium antagonist to be localized in the diseased site without adversely affecting the patient or releasing excessive amounts of the drug into the circulation system. If a steroid is used following, the calcium antagonist, the acceptable dose of steroid may be administered through several methods. The Steroid, either alone or in combination with a pharmaceutically acceptable vehicle, can be topically applied to the surface of the wound site; injected into the wound site; or incorporated into a controlled release polymer and surgically implanted into the region to be treated.

Depolymerization of cycloskeletal proteins leading to alteration of cell shape and matrix degradation can be regulated by this invention. Secondary to this, the invention can be used to regulate and block exocytosis. In particular, fibroblasts are contacted with an effective amount of a calcium antagonist sufficient to degrade the matrix and retard exocytosis to a desired degree. The method ot contacting the calcium antagonists to the fibroblast cells of interest and the effective amount of these drugs are described above.

In addition to treating hypertrophic wound healing disorders, one or combinations of several different calcium antagonists co-adminiszered to a patient can be used to therapeutically control diseases caused by excessive fibroblast biosynthesis. Diseases characterized by fibroblast overproduction include, cirrhosis of the liver, constrictive pericarditis, Dupuytren's disease of the hand, as well as other fibromatoses. While these are but a few diseases which can be treated with the aid of this invention, it should be recognized that any disease which is characterized by excessive fibroblast biosynthesis can be treated using calcium antagonists.

The invention is further illustrated by the following Example which should not be construed to be limiting in any way:

### Example 1

### Studies on Protein and GAG Secretion

### Tissue Preparation

A connective tissue model of uniaxially oriented cells and extracellular matrix was fabricated using bovine fibroblasts, rat tail tendon collagen and nutrient media. Bovine fascial fibroblasts were harvested from the thigh of freshly slaughtered 2 week old calves (Trelegan's, Cambridge, MA) by enzymatic digestion using 0.1% type II collagenase (Worthington Biochemical, Inc., Freehold, NJ) digestion in Dulbecco's Modified Eagle Medium (DMEM; Gibco, Grand Island, NY) at 37°C for 4 hours. The released cells were seeded onto tissue culture dishes with DMEM supplemented with 10% NuSerum™ (Collaborative Research, Bedford, MA). The media was changed every 48 hours. The cells were passaged once and then either used immediately or stored frozen in 50% calf serum/45% DMEM/5% DMSO at -100'C. When frozen cells were used they were quickly thawed, sedimented through a column of 50% serum/50% DMEM at 185g for 3 minutes, then plated on coverslips. The media was changed after cell attachment (-4 hours).

Native type I collagen was extracted from rat tail tendon and purified using a modification of the method of Chandrakasan, G. et al., J. Biol. Chem. 251: 6062-6067 (1976). Specifically, rat tail tendons were removed from adult Sprague-Dawley rat tails, washed in PBS and distilled water. The tendons were then placed in a solution of 0.05M (3%) acetic acid at the ratio of 200 ml per tail. The mixture was stirred for 96 hours at 8°C.

After 24 hours of stirring, the mixture was filtered through several layers of cheese cloth and then centrifuged at 12000g (9000 rpm in Sorval GS-3 rotor) for 2 hours. The supernatant was precipitated and redissolved in cold acid multiple times to remove non-collagenous proteins. The collagen solution was sterilized in 1/1000 (v/v) chloroform. This procedure preserves the native structure of the collagen molecule.

Oriented tissue equivalents were made by mixing bovine fibroblasts with a 0.2% collagen solution, 20% calf serum, 10mg/ml gentamycin solution, 5 mg/ml ascorbate in DMEM as previously described [McLeod, K.J. "Modulation of Biosynthesis by Physiologically Relevant Electric Fields" Ph.D. Thesis M.I.T. 1986]. This suspension was poured into sterile culture dishes containing two sterile porous polyethylene posts held 2 centimeters apart. The dishes were placed in a cell culture incubator gassed at 5% CO₂ with 99% humidity. The suspension gelled at 37°C.

over several days, the fibroblasts remodelled and consolidated the collagen gel around the fixed posts. The resultant oriented fibroblast populated collagen matrix (FPCM) formed a tissue equivalent structure which histologically resembled a ligament. oriented tissue equivalents are further described in McLeod et al., Science 236: 1465-1469 (1987) and in U.S.-A-5,521,087 (Application Serial No. 07/349,855, filed May 10, 1989) to R.C. Lee and D. Huang.

### Assay for Determining Biosynthetic Activity

Protein and glycosaminoglycan (GAG) biosynthesis was measured using radiolabeled precursors of protein and sulfated GAGs. Four days after casting of the FPCM, the media bathing the ligament equivalents was changed to serum free DMEM with 0.5mM L-proline (Sigma, St. Louis, MO). After 12 hours, the media was changed again to fresh serum-free media supplemented with 10 µCi/ml Na₂³⁵SO₄ (NEX-041, New England Nuclear, Boston, MA), 10 µCi/ml L-[5-³H]-proline (NET-573, New England Nuclear, Boston, MA) and 0.5 mM L-proline. The samples were bathed in the radiolabeled media for 12 hours. The radiolabeled sulfate was incorporated into GAGs and ³H-proline into proteins, and so provided markers of sulfated GAGs and protein synthesis, respectively. Since DMEM is proline-free, the addition of non-radioactive proline ensures a relatively constant specific activity in the medium.

### Effect of Calcium Antagonists on Biosynthesis

The effect of calcium antagonists on protein and glycosaminoglycan (GAG) biosynthesis was measured in FPCMs under several conditions. The biosynthetic responses to calcium antagonism were studied in FPCMs cultured in DMEM supplemented with either 5.5 µM glucose or 5.5 µM fructose. Both were studied because energy metabolism of cultured fibroblasts is primarily anaerobic when the carbohydrate energy source is glucose and predominantly aerobic when the carbohydrate source is fructose [Thilly, W.G., Mammalian Cell Technology, Chapter 5, Butterworth Publishers, Boston, (1986)]. In vivo fibroblasts are, however, believed to derive their energy primarily through aerobic glycolysis.

The drugs used to antagonize calcium channels were verapamil, nifedipine, cobalt chloride and the calmodulin inhibitor, trifluoperazine. Control studies were performed to test the metabolic state of the cells in the FPCM. The effect of hydroxyurea and antimycin A, a drug which blocks oxidative phosphorylation, on biosynthesis was measured in FPCMs cultured in fructose or glucose. Use of verapamil, nifedipine and cobalt chloride for the manufacture of medicaments and compositions for scar treatment is outside the scope of the presently claimed invention. fibroblasts provided with glucose or fructose as energy substrates were observed. The effects of antimycin A on both incorporation of proline into extracellular matrix protein and incorporation of sulfate into extracellular matrix glucosaminoglycans over a 12 hour period was measured in FPCMs bathed in DMEM/5.5 mM fructose and their results are shown in Figures 1a and 1b, respectively. Antimycin A had little effect on ³H-proline incorporation with FPCMs provided with glucose. In contrast, antimycin A caused a substantial reduction in the rate of proline incorporation into the extracellular matrix in FPCMs provided with fructose.

## Claims

1. The use of a calmodulin inhibitor alone or in admixture with a pharmaceutically acceptable vehicle, for the manufacture of a medicament for controlling scar production in the skin, and reducing any preexisting scar tissue in the skin.

2. The use of a composition comprising a calmodulin inhibitor alone or in admixture with a pharmaceutically acceptable vehicle, and a protein synthesis inhibitor or vitamin E for the manufacture of a medicament formulation for controlling scar production at a wound site in the skin and reducing any preexisting scar tissue at the wound site, said calmodulin inhibitor and said protein synthesis inhibitor or Vitamin E being sequentially administrable.

3. The use according to claim 1 or 2, wherein the calmodulin inhibitor is trifluoperazine.

4. The use according to claim 1 or 2, wherein the medicament is adapted for administration by injection into the wound site, topical application onto the scar site or by incorporation into a release-controlling polymer.

5. The use according to claim 2, wherein the protein synthesis inhibitor is a corticosteroid or glucocorticosteroid such as triamcinolone acetonide.

6. The use of a calmodulin inhibitor for the manufacture of a medicament for controlling scar production in the skin associated with a hypertrophic wound healing disorder or burn scar contracture.

7. The use according to claim 6, wherein the calmodulin inhibitor is trifluoperazine.

8. The use of claim 2, wherein the protein synthesis inhibitor is a steroid.

9. The use of claim 8, wherein the steroid is a corticosteroid or glucocorticosteroid.

10. The use, alone or in admixture with a pharmaceutically acceptable vehicle, of a calcium antagonist consisting of a Protein Kinase C inhibitor as the active ingredient, for the manufacture of a medicament for controlling scar production in the skin and reducing any preexisting scar tissue in the skin.

11. The use of a calcium antagonist consisting of a Protein Kinase C inhibitor as the active ingredient and a pharmaceutically acceptable vehicle, in combination with a protein synthesis inhibitor or Vitamin E for the manufacture of a medicament formulation, for controlling scar production at a wound site in the skin and reducing any preexisting scar tissue at the wound site, said antagonist and protein synthesis inhibitor or Vitamin E being administrable sequentially.

12. The use as the active ingredient, of a calcium antagonist consisting of a Protein Kinase C inhibitor, for the manufacture of a medicament for controlling scar production in the skin associated with hypertrophic wound healing disorder or burn scar contracture.

13. The use according to claim 8, 9 or 10, wherein the Protein Kinase C inhibitor is tamoxifen.

14. The use according to any of claims 1, 2, 10 and 11, wherein said scar is associated with a fibromatosis, keloid or a hypertrophic wound healing disorder.

## Patentansprüche

1. Verwendung eines Calmodulin-Inhibitors allein oder im Gemisch mit einem pharmazeutisch akzeptablen Vehikel zur Herstellung eines Medikaments zur Steuerung der Narbenproduktion in der Haut und zur Reduktion irgendwelcher vorher vorhandener Narbengewebe in der Haut.

2. Verwendung einer Zusammensetzung umfassend einen Calmodulin-Inhibitor allein oder im Gemisch mit einem pharmazeutisch akzeptablen Vehikel, und einen Proteinsynthese-Inhibitor oder Vitamin E für die Herstellung einer Medikamentenformulierung zur Steuerung der Narbenproduktion an einer Wundstelle in der Haut und zur Reduktion irgendwelcher vorher vorhandener Narbengewebe an der Wundstelle, wobei dieser Calmodulin-Inhibitor und dieser Proteinsynthese-Inhibitor oder dieses Vitamin E der Reihe nach verabreichbar sind.

3. Verwendung nach Anspruch 1 oder 2, wobei der Calmodulin-Inhibitor Trifluoperazine ist.

4. Verwendung nach Anspruch 1 oder 2, wobei das Medikament für die Verabreichung durch Injektion in die Wundstelle, die topische Anwendung auf die Stelle der Narbe oder den Einschluss in ein die Freisetzung steuerndes Polymer hergerichtet ist.

5. Verwendung nach Anspruch 2, wobei der Proteinsynthese-Inhibitor ein Corticosteroid oder Glucocorticosteroid, wie Triamcinolonacetonid ist.

6. Verwendung eines Calmodulin-Inhibitors zur Herstellung eines Medikaments zur Steuerung der Narbenproduktion in der Haut im Zusammenhang mit einer hypertrophen Wundheilungsstörung (hypertrophic wound healing disorder) oder der Kontraktur verbrannter Haut.

7. Verwendung nach Anspruch 6, wobei der Calmodulin-Inhibitor Trifluoperazine ist.

8. Verwendung nach Anspruch 2, wobei der Proteinsynthese-Inhibitor ein Steroid ist.

9. Verwendung von Anspruch 8, wobei das Steroid ein Corticosteroid oder Glucocorticosteroid ist.

10. Verwendung eines Calciumantagonisten, bestehend aus einem Protein-Kinase-C-Inhibitor als aktivem Inhaltsstoff, alleine oder im Gemisch mit einem pharmazeutisch akzeptablen Vehikel, zur Herstellung eines Medikaments zur Steuerung der Narbenproduktion in der Haut und zur Reduktion irgendwelcher vorher vorhandener Narbengewebe in der Haut.

11. Verwendung eines Calciumantagonisten, bestehend aus einem Protein-Kinase-C-Inhibitor als aktivem Inhaltsstoff und einem pharmazeutisch akzeptablen Vehikel, in Verbindung mit einem Proteinsynthese-Inhibitor oder Vitamin E zur Herstellung einer Medikamentenformulierung zur Steuerung der Narbenproduktion an einer Wundstelle in der Haut und zur Reduktion irgendwelcher vorher vorhandenen Narbengewebe an der Wundstelle, wobei dieser Antagonist und dieser Proteinsynthese-Inhibitor oder dieses Vitamin E der Reihe nach verabreichbar sind.

12. Verwendung eines Calciumantagonisten als aktivem Inhaltsstoff, bestehend aus einem Protein-Kinase-C-Inhibitor, zur Herstellung eines Medikaments zur Steuerung der Narbenproduktion in der Haut im Zusammenhang mit einer hypertrophen Wundheilungsstörung (hypertrophic wound healing disorder) oder der Kontraktur verbrannter Haut.

13. Verwendung nach Anspruch 8, 9 oder 10, wobei der Protein-Kinase-C-Inhibitor Tamoxifen ist.

14. Verwendung nach einem der Ansprüche 1, 2, 10 und 11, wobei diese Narbe im Zusammenhang steht mit einer Fibromatose, einem Keloid oder einer hypertrophen Wundheilungsstörung (hypertrophic wound healing disorder).

## Revendications

1. Utilisation d'un inhibiteur de la calmoduline seul ou en mélange avec un véhicule pharmaceutiquement acceptable, pour la fabrication d'un médicament destiné à contrôler la production de cicatrice sur la peau, et à réduire tout tissu cicatriciel préexistant sur la peau.

2. Utilisation d'une composition comprenant un inhibiteur de la calmoduline seul ou en mélange avec un véhicule pharmaceutiquement acceptable, et un inhibiteur de la synthèse des protéines ou de la vitamine E, pour la fabrication d'une formulation médicamenteuse destinée à contrôler la production de cicatrice au site de la plaie sur la peau, et à réduire tout tissu cicatriciel préexistant au site de la plaie, ledit inhibiteur de la calmoduline et ledit inhibiteur de la synthèse des protéines ou la vitamine E pouvant être administrés séquentiellement.

3. Utilisation selon la revendication 1 ou 2, dans laquelle l'inhibiteur de la calmoduline est la trifluopérazine.

4. Utilisation selon la revendication 1 ou 2, dans laquelle le médicament est adapté pour une administration par injection dans le site de la plaie, par application topique sur le site de la cicatrice ou par incorporation dans un polymère de contrôle de la libération.

5. Utilisation selon la revendication 2, dans laquelle l'inhibiteur de la synthèse des protéines est un corticostéroïde ou un glucocorticostéroïde tel que l'acétonide de triamcinolone.

6. Utilisation d'un inhibiteur de la calmoduline pour la fabrication d'un médicament destiné à contrôler la production de cicatrice sur la peau, associée à un trouble de la cicatrisation hypertrophique des plaies ou à une contraction de cicatrice de brûlure.

7. Utilisation selon la revendication 6, dans laquelle l'inhibiteur de la calmoduline est la trifluopérazine.

8. Utilisation selon la revendication 2, dans laquelle l'inhibiteur de la synthèse des protéines est un stéroïde.

9. Utilisation selon la revendication 8, dans laquelle le stéroïde est un corticostéroïde ou un glucocorticostéroïde.

10. Utilisation, seul ou en mélange avec un véhicule pharmaceutiquement acceptable, d'un inhibiteur calcique consistant en un inhibiteur de la protéine kinase C en tant que principe actif, pour la fabrication d'un médicament destiné à contrôler la production de cicatrice sur la peau, et à réduire tout tissu cicatriciel préexistant sur la peau.

11. Utilisation d'un inhibiteur calcique consistant en un inhibiteur de la protéine kinase C en tant que principe actif, et d'un véhicule pharmaceutiquement acceptable, en combinaison avec un inhibiteur de la synthèse des protéines ou la vitamine E pour la fabrication d'une formulation médicamenteuse destinée à contrôler la production de cicatrice au site de la plaie sur la peau, et à réduire tout tissu cicatriciel préexistant au site de la plaie, lesdits inhibiteur calcique et inhibiteur de la synthèse des protéines ou la vitamine E pouvant être administrés séquentiellement.

12. Utilisation comme principe actif, d'un inhibiteur calcique consistant en un inhibiteur de la protéine kinase C, pour la fabrication d'un médicament destiné à contrôler la production de cicatrice sur la peau, associée à un trouble de la cicatrisation hypertrophique des plaies ou à une contraction de cicatrice de brûlure.

13. Utilisation selon la revendication 8, 9 ou 10, dans laquelle l'inhibiteur de la protéine kinase C est le tamoxifène.

14. Utilisation selon l'une quelconque des revendications 1, 2, 10 et 11, dans laquelle ladite cicatrice est associée à une fibromatose, une chéloïde ou un trouble de la cicatrisation hypertrophique des plaies.
